# EUROPEAN PATENT SPECIFICATION

(11) **EP 1 946 788 B1**
(45) Date of publication and mention of the grant of the patent: **15.09.2010**
(21) Application number: 08004372.2
(22) Date of filing: 01.10.2004
(51) Int. Cl.: A61M 5/28, A61B 17/34

(54) **Syringe for administering a therapeutic agent into tissue**
Spritze zur Verabreichung eines therapeutischen Mittels in Gewebe
Seringue pour administrer un agent thérapeutique dans un tissu

(30) Priority: 06.10.2003 US 508300 P; 15.06.2004 US 867215
(43) Date of publication of application: 23.07.2008
(62) Divisional of application: 04791785.1
(73) Proprietor: Activeo, LLC, Salt Lake City, UT 84095 (US)
(72) Inventor: Murphy, Kieran P. Dr., Baltimore, MD 21210 (US); Muto, Mario, I-80122 Napoli (IT)
(74) Representative: Pawlyn, Anthony Neil

(56) References cited:
- DE-C- 447 936
- US-A- 5 971 722

## Description

### FIELD OF THE INTENTION

The present invention relates generally to an apparatus for administering a therapeutic agent into tissue and in particular, for the administration of a therapeutic agent such as medical ozone.

### BACKGROUND OF THE INVENTION

Back joint disc or tendon pain is a common and potentially debilitating ailment that affects an estimated 80% of the worldwide population at least once in a lifetime. In many instances, the cause of the pairi can be attributed to a degenerated intervertebral disc that has further deteriorated into a condition known as disc herniation This occurs when the disc nucleus pulposus extrudes through a tear or fissure in the outer lining of the disk, thereby exerting pressure on spinal nerves. The compression caused by the herniated nucleus leads to inflammation and is directly responsible for the pain felt down the leg (also referred to as sciatica). Available treatments for this type of back pain vary according to the severity of the hernia. If mild, the patient's condition can be appeased with rest and inactivity for an extended period of time. However, for patients suffering from a severe herniation or who do not respond to non-invasive treatment (pharmacological and/or physical therapy), surgical intervention is often recommended. With this invasive treatment come several disadvantages such as:
i) irreversibility of the procedure
ii) formation of scar tissue
iii) slower recovery time
iv) longer hospital stays

Since the late 1950s, many attempts have been made to treat sciatica and lower back pain with percutaneous procedures to avoid surgery. Well known treatments for example are percutaneous discetomy and chemonucleolysis but the cost of these procedures has kept researchers looking for another alternative. It was in 1984 that an Italian orthopedic surgeon by the name of Dr. Cesare Verga first proposed the use of ozone/oxygen mixtures to treat the pathology of a herniated disk. (See for example, http://www.cleanairassociationlcom/6/ca_3.htm, Ozone Therapy: New breakthrough for Back Treatment, by Gaetano Morello, M.D., the contents of which are incorporated herein by reference.)

Other prior art references include: Percutaneous Treatment of Herniated Lumbar Disc by Intradiscal Oxygen-Ozone Injection, M. Muto and F. Avella, Interventional Neuroradiology 4:279-286, 1998.

In other situations such as rheumatoid arthritis, osteoarthritis or a repetitive injury through sports or occupation, such as tennis elbow, frozen shoulder, or house maids knee, inflammation can develop between two surfaces which are involved in allowing joint function, such as a tendon and the sheath or lubricated tube in which that tendon moves. Inflammation such as bursitis in the knee shoulder hip, or other anatomic bursa may benefit from ozone therapy, this includes epicondylitis, and other tendonitis and bursitis, including the wrist, hand and the tendon sheaths of the hand and wrist. Inflammation can occur at a site where a tendon or a ligament insert to bone or pass through a sheath from trauma, tension, over use or disease.

Inflammation can develop through pathologies of any joint, and these may again include the inflammatory arthropatic conditions of rheumatoid arthritis, psoriatic arthritis and the like, or osteoarthritis. Joints that may be involved in these processes that are amenable to ozone injection include the synovial joints such as the, temperomandibular joint, the hip joint, knee joint, ankle joint, elbow joint or sacro-iliac joint. Vertebral facet and sacro-iliac joints may also benefit, inflammatory involvement of joints in the hand, wrist and feet with rheumatoid arthritis, osteoarthritis or a repetitive injury through sports or occupational such as carpal tunnel syndrome.

The inflammatory and arthritic or degenerative discussions described above are usually treated with a combination of anti-inflammatory agents such as ibuprofen, or more powerful drugs such as steroids or chemotherapy such as methotrexate. It is a common medical practice to inject steroid medications or lidocaine directly into the inflamed tissue or joint. This is often done repeatedly. These drugs can be associated with side effects of infection and even death from gastric ulcer bleeding or immunosurpression and infection. We believe that ozone therapy whether as a liquid or a gas would have advantages over the current practice.

Lavage of a surgical space prior to placement of a permanent surgical implant such as a hip or knee prosthesis, or pacemaker or treatment of an infected joint can be facilitated by the use of medical ozone as a sterilizing substance. Similarly a colostomy stoma can be created such that the adhesive disk is infused with a ozone as a liquid or gas to aid in healing and inhibit infection. The post surgical recovery from sternotomy after cardiac surgery is often complicated wound infection. Placement of a resorbable catheter in the wound that could be irrigated with ozone liquid or gas would aid healing. Indeed any wound could have a resorbable multisided hole catheter placed in it to allow ozone be injected through it. This would have anti-infective, analgesic and promote wound healing properties. This would shorten recovery time and decrease complication rates after surgery.

Enhance liquid ozone could be applied to the wound /surgical site healing at a site of high probability of infection such an abdominal incision/wound after appendectomy, or urgent colectomy with colostomy or after percutaneous endoscopic cholecystectomy.

Endoscopic procedural infusion of ozone and trans catheter infusion of ozone can be used to inhibit the complications endoscopic medical intervention or image guided or non-image guided catheter based intervention for example in endoscopic evaluation of the pancreatic duct.

Dental injection of liquid or gas ozone may augment the preparation and repair of dental cavities, and aid in reduction of root canal inflammation or periodontal disease.

There are veterinary applications of minimally invasive ozone administration in animals diseased with disc and degenerative syndromes. Few other options are available in that arena. Some animals are destroyed due to debilitating pain secondary to pain from disc disease, and arthritis.

While the full therapeutic potential of ozone continues to unfold with ongoing research, it is already clear that this form of therapy for the treatment of disc herniation has significant advantages over other surgical and percutaneous procedures. Some of these advantages include:
- fewer clinical and neuroradiological contraindications
- success rates greater than 70% in the intervertebral disc
- little or no recovery time
- little or no side effects
- little or no scar tissue formed
- minimally invasive procedure
- effective alternative treatment for which response to conservative management, such as rest and reduced daily activity, has failed to treat
DE447936 provides an ozone gas generator according to the preamble of the claimed invention, but provided with an external power supply.

As the success of ozone gas therapy continues to gain recognition in the medical arena as a non-invasive alternative for the treatment of disc herniation, current methods of administering an effective dose of the ozone are solely as a gas and are far from optimum. There also lacks a sterile methodology through which the ozone can be delivered selectively to the pain-affected area, i.e. the herniated disk. The gas is unstable with a half life measured in seconds. There are no dedicated medical ozone generators that are disposable single use units. In accordance with this, there is a need for equipment especially designed for the treatment of disc herniation and other medical conditions affecting the body with medical ozone so that it can be done in an efficient and sterile manner. There is a need to develop kits for intervention in inflammatory and degenerative disease, that are disposable, or reusable, but aid in creating sterile, stable, ozone rapidly on demand. The generation of ozone liquid from sterile water would allow storage of injector/generators in all medical dental and veterinary facilities.

### SUMMARY OF THE INVENTION

It is therefore an object of the present invention to provide a novel apparatus for administering a gas into a tissue that obviates or mitigates at least one of the disadvantages of the prior art.

In a first aspect of the invention there is provided a syringe for self-contained generation and administration of a therapeutic agent comprising:
a barrel for holding said agent;
a plunger for insertion into a first end of said barrel and expressing said agent from a second end of said barrel;
a power supply integrally mounted coaxially with said plunger;
a therapeutic agent generator integrally mounted coaxially with said plunger and in communication with said barrel through a channel in said plunger, said generator connected to said power supply and mounted coaxially with said plunger; a switch connected to said power supply such that when said switch is activated said therapeutic agent generator generates said therapeutic agent and fills said barrel therewith.
The syringe may be a component of a kit for administering therapeutic gas mixture into soft tissue, wherein said gas mixture includes ozone. The kit can comprise of the following items:
- Disinfectant
- Drape
- Skin preparation material
- Local anaesthetics contained in vials
- Syringes
- Short and long needles, some with side holes
- Gantry drape
- Radiolucent needle holder
- Nonionic x-ray dye-for discogram
- Infusion wire
- Charcoal ozone scavenger
- Steroids contained in vials
- A source of ozone either generated as a gas or a liquid in a disposable single use unit that is sterilizable. The ozone can be generated in the delivery system as a battery powered electronic
- hand held disposable device
- The ozone generator based in the injector will be available in a
- variety of sizes, capable of delivering a range of ozone volumes, from 1 cc to 5 cc, but could be made to generate volumes of ozone liquid or gas form 0.1 cc to 1 litre.
- It is probable that the commonly used volumes would be between 1 cc and 10 cc allowing for dead space in the connecting tubing/needle etc.
- Disposable filtered attachment
- Stop cocks
- Non-compliant tubing
- Post-operative dressing for skin closure
- Adhesive bandages with gauze pad in the centre

An example of a local anaesthetic is, but not limited to, Lidocaine Hcl

An example of a nonionic x-ray dye is, but not limited to, Omnipaque 300 M.

An example of a post-operative dressing is, but not limited to, Povidone gel.

Any suitable source of ozone can be used such as an ozone generator, the AOS-1M Medical Ozonator or AOS-IMS Stainless Medical Ozonator for example, or a disposable injector filled with ozone.

The invention may be used in a method for the treatment of pain caused by a herniated disc and comprising of first identifying the herniated disk using an imaging technique and subsequently injecting a known volume of medical ozone into the disc and paraspinous soft tissues in a sterile manner and environment.

The invention may be used in an apparatus for administering a therapeutic agent comprising a therapeutic agent generator and a scavenger connected to the generator via a first valve for capturing the therapeutic agent. The therapeutic agent administrator is connectable to the generator via at least one additional valve such that when the valves are in a first position the generator communicates with the administrator for filling the administrator with the agent. When the valves are in a second position the administrator retains the agent therein upon disconnection from the generator, and the scavenger captures any excess agent intermediate the administrator and the generator.

The agent can be gaseous or liquid ozone. The generator can be a medical ozone generator. The medical ozone can be a ratio of oxygen (02) and ozone (03).

The ratio of oxygen to ozone can be about 1µg/ml, or about 10µg/ml, or about 20µg/ml, or about 30µg/ml, or about 40µg/ml, or about 50µg/ml.

The ratio of oxygen to ozone can be between about 1µg/ml and about 90µg/ml. The ratio of oxygen to ozone can be between about 10µg/ml and about 80µg/ml. The ratio of oxygen to ozone can be between about 20µg/ml and about 70µg/ml. The ratio of oxygen to ozone can be between about 10µg/ml and about 34µg/ml. More preferably, The ratio of oxygen to ozone can be between about 27µg/ml to about 28µg/ml.

The administrator can be a syringe.

The invention may be used in a method of treating the pain caused by a herniated disc comprising identifying the herniated disk and injecting medical ozone into the disc and paraspinous soft tissues.

The invention may provide that the ozone can be generated either as a liquid or a gas, in a syringe type structure where the electronics are housed in the area normally occupied by the plunger of the syringe and the anode is in the syringe. The syringes can be any desired volumes, such as those ranging from 1 cc to 60 cc, more preferably 1cc to 10 ccs. The syringes are typically made of polyethylene to resist the corrosive effect of ozone even in the short time it in contact with the plastic.

The invention may be used in providing an implantable apparatus for self-contained generation and administration of a therapeutic agent comprising: a chamber for holding the agent and a catheter for connecting the chamber to an area for administration of the agent. The apparatus also includes a power supply and a therapeutic agent generator in communication with the chamber, the generator connected to the power supply. The apparatus also includes a switch activateable externally by a patient implanted with the apparatus. The switch is connected to the power supply such that when the switch is activated the therapeutic agent generator generates the therapeutic agent and fills chamber therewith.

The invention may be used in providing a method of treating pain caused by a herniated disc comprising of the following steps:
identifying the herniated disk with an imaging device;
preparation of the skin above the affected area;
applying local anaesthetics to the operation site;
inserting a needle down to the disc level at the herniated disc;
injecting nonionic x-ray dye down the needle;
performing a discogram of the disc;
activating an ozone generator external to the patient;
selecting a specific concentration of O3/O2 gas mixture on the ozone generator;
attaching a scavenger and a syringe to the ozone generator;
aspirating the O3/O2 gas mixture into the syringe;
injecting a predetermined volume of a fixed concentration of ozone/oxygen gas mixture into the disc and paraspinous soft tissues of the disc;
capturing unused ozone in the charcoal scavenger; and,
removing all needles;
dressing the operative site.

### BRIEF DESCRIPTION OF THE DRAWINGS

Preferred embodiments of the invention and subject matter useful for the understanding of the invention will now be discussed, by way of example only, with reference to the attached Figures, in which:
Figure 1 shows an apparatus for administering a gas into a tissue and which is useful for understanding the present invention;
Figure 2 shows the hip area of a patient where a therapeutic agent can be administered;
Figure 3 shows the spinal disc area of a patient where a therapeutic agent can be administered;
Figure 4 shows a normal spinal disc;
Figure 5 shows a herniated spinal disc where a therapeutic agent can be administered;
Figure 6 shows an apparatus for administering a gas into a tissue and which is useful for understanding the present invention;
Figure 7 shows the administrator of Figure 6 with the needle placed thereon;
Figure 8 shows an apparatus for administering a gas into a tissue in accordance with an embodiment of the invention;
Figure 9 shows an apparatus for administering a therapeutic agent into a hip region and which is useful for understanding the present invention;
Figure 10 shows the apparatus of Figure 9 in greater detail;
Figure 11 shows an infusion wire through which a therapeutic agent can be administered;
Figure 12 shows the infusion wire being passed through a needle towards the centre of a herniated disc;
Figure 13 shows the infusion wire being placed in centre of a herniated disc with the needle having been removed;
Figure 14 shows the infusion wire of Figure 11 in greater detail; and,
Figure 15 shows an apparatus for administering a therapeutic agent into a tissue.

### DETAILED DESCRIPTION OF THE INVENTION

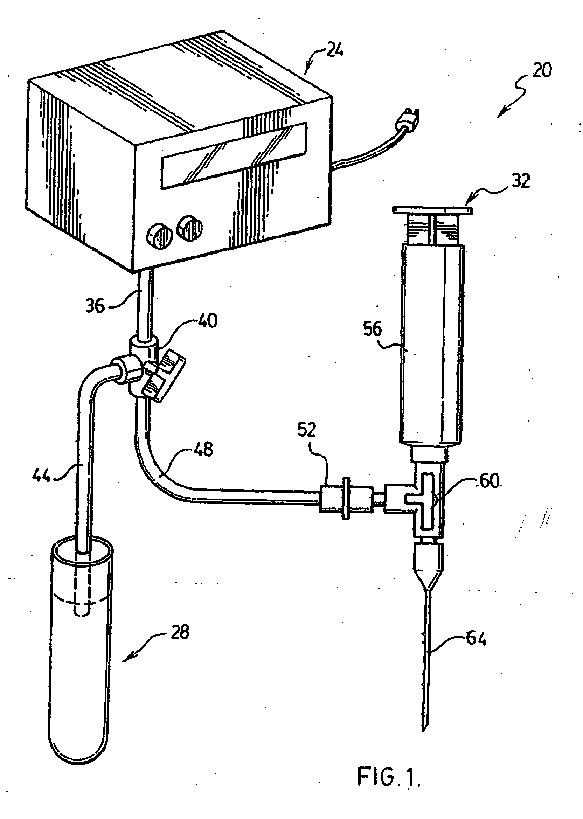

Referring now to Figure 1, an apparatus for administering a therapeutic agent, such as an ozone gas, into a tissue is indicated generally at 20. The apparatus 20 comprises an ozone generator 24 that connects to a charcoal scavenger 28 and an ozone administrator 32. Generator 24 can be based on any known medical ozone generator. Generator 24 connects to scavenger 28 and administrator 32 via a network of flexible tubing and valves for selectively directing the flow of gas there between. More specifically, a first segment of tubing 36 connects generator 24 to a three-way valve 40. A second segment of tubing 44 connects valve 40 to scavenger 28. A third segment of tubing 48 connects valve 40 to another valve 52, which in turn connects to administrator 32. The tubing is made of any suitable material, such as silicon of the known medical type, and has a diameter and wall thickness to withstand the pressure of ozone gas being carried therethrough. The valves, also known as stopcocks, are also of the known medical type and have fittings complementary to the various portions of tubing.

Administrator 32 is comprised of a syringe 56, a three-way valve 60, and a needle 64. Syringe 56 is typically made of polyethylene to resist the corrosive effect of ozone, but other materials will occur to those of skill in the art. Three-way valve 60 is releasably connectable directly to valve 52, providing a selective pathway between syringe 56 and generator 24 and/or scavenger 28.

Valve 40 has a first position wherein generator 24 communicates only with tubing 48. Valve 52 has a first position wherein tubing 48 communicates with administrator 32. Valve 60 has a first position wherein syringe 56 communicates only with tubing 48. Thus, when valves 40, 52 and 60 are all in the first position, generator 24 is in communication with syringe 56, and thus when generator 24 is "on", syringe 56 will be filled with ozone. (It is to be noted that in of the Figures attached hereto, for this illustration and subsequent illustrations and embodiments, the various valves depicted therein are not intended to be shown in any specific position, and are merely illustrated to show their physical orientation in relation to the rest of the components in the apparatus.)

Valve 40 has a second position wherein tubing 44 communicates with both tubing 36 and 48. Valve 52 has a second position wherein tubing 48 is effectively capped, preventing communication between administrator 32 and tubing 48. When valves 40 and 52 are in the second position, any ozone generated by generator 24 is captured by scavenger 28. The scavenger 28 is of the charcoal type, but any type of scavenger for capturing excess ozone can be used.

Once generator 24 is turned "off" after filling syringe 56 as described above, then when valves 40 and 52 are each moved from their respective first position to their respective second position then any excess ozone still present in tubing 48 and 36 will be captured by scavenger 28 and thereby reduce and/or substantially eliminate the unwanted escape of ozone into the atmosphere where it may harm the operator or other individuals proximal to apparatus 20.

Valve 60 also has a second position wherein syringe 56 is prevented from communicating with either valve 52 (or the open fitting on valve 60 that connects to valve 52), or with needle 64. Thus, once syringe 56 has been filled with ozone, leaving administrator 32 'charged' with ozone, valve 60 will also be placed in its second position to retain the ozone within syringe 56 once administrator 32 is disconnected from valve 52.

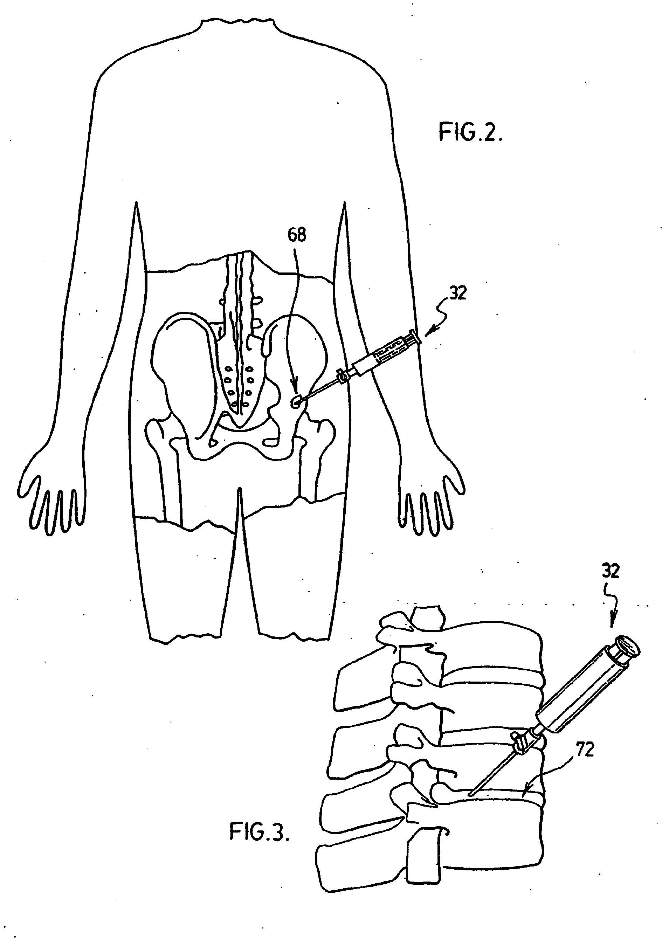

Once administrator 32 is charged with ozone, it can be disconnected from the remainder of apparatus 20 so that it can be used to administer ozone to a target area. Thus, valve 60 also has a third position that places syringe 56 in communication with needle 64. Referring now to Figure 2, when charged, valve 60 can be placed in the second position to prevent ozone from escaping from syringe 56. Administrator 32 can then be disconnected from the remainder of apparatus 20 and then needle 64 can be inserted into tissue (or other target area) where the ozone is to be administered. In Figure 2, needle 64 is shown inserted into a hip 68. Having inserted needle 64 into hip 68, valve 60 is placed in the third position, allowing syringe 56 to communicate with needle 64. At this point syringe 56 is depressed, and the ozone gas therein is expressed out of needle 64 into hip tissue, thereby providing localized pain relief around hip 68. Such administration of ozone can be helpful to relieve pain after some types of hip surgeries, such as hip replacement, or after bone is harvested from the pelvis to use as a bone graft material.

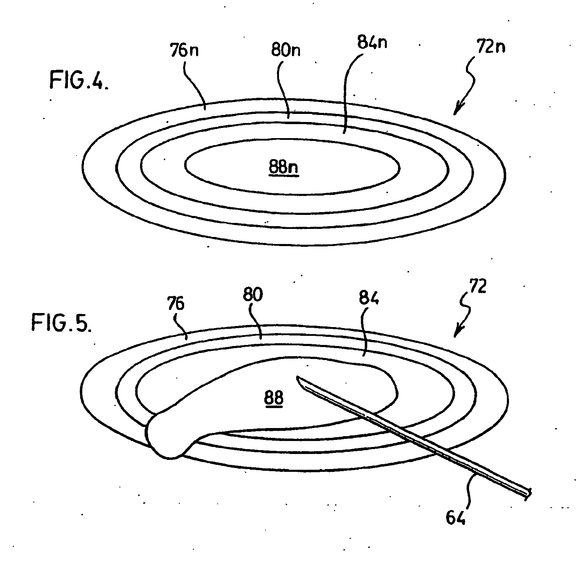

Other types of localized pain relief can also be provided. While Figure 2 depicts pain relief being provided to hip 68, Figure 3 depicts the provision of pain relief to a spinal disc 72. By way of background, as seen in Figure 4 a normal disc is shown in cross section at 72n. Disc 72n has an outer annulus fibrosus 76n, an inner annulus fibrosus 80n, a transition zone 84n, and a nucleus pulposus 88n. However, in Figure 5, disk 72 is also shown in cross section, wherein nucleus pulposus 88 is protruding, and thereby a cause of pain. However, the administration of ozone into the protruding nucleus pulposus 88 using administrator 32 via needle 64 pain can be relieved and/or substantially eliminated at least temporarily.

It should now be apparent that having administered ozone from administrator 32, administrator 32 can then be reconnected to the remainder of apparatus 20, and the appropriate valves 40, 50 and 62 adjusted to either allow any remaining ozone to be expressed from administrator 32 for capture by scavenger 28, or to refill syringe 56.

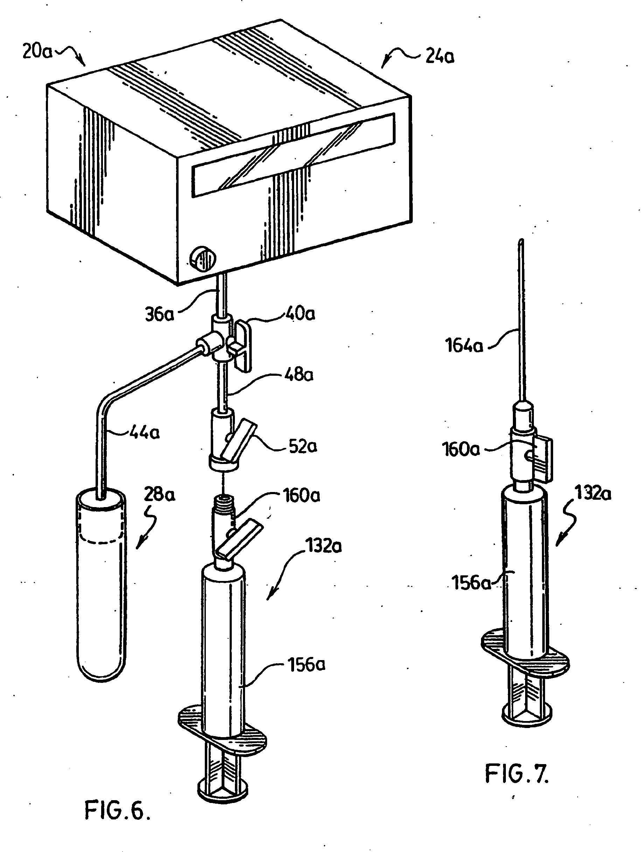

Referring now to Figure 6, an apparatus for administering a gas into a tissue is indicated generally at 20a. Apparatus 20a includes many similar components to apparatus 20, and like components are indicated with like references but followed by the suffix "a". In contrast to apparatus 20, however, administrator 132a includes certain differences from administrator 32. Specifically, administrator 132a has a two way valve 160a, one end of which connects to syringe 132a, the other end of which has a fitting to allow administrator 132a to be connected to either valve 52a, as shown in Figure 6, or to needle 164a as shown in Figure 7. When administrator 132a is connected to valve 52a as shown in Figure 6, valves 52a and 160a can be placed in an open position so that syringe 132a communicates with generator 24a. When generator 24a is "on", syringe 132a will be filled with ozone. Once syringe 132a is filled with ozone, valves 52a and 160a can be placed in the closed position, and valve 40a can be turned so that once apparatus 160a is disconnected from valve 52a, any ozone remaining in tubes 48a, 36a or elsewhere in that remaining portion of apparatus 24a can be captured by scavenger 28a.

Having filled syringe 132a with ozone, and closed valve 160a, needle 164a can then be affixed thereto as shown in Figure 7. Valve 160a can then be selectively opened or closed to allow the administration of ozone to tissue, in much the same manner as previously described in relation to Figures 2 and 3.

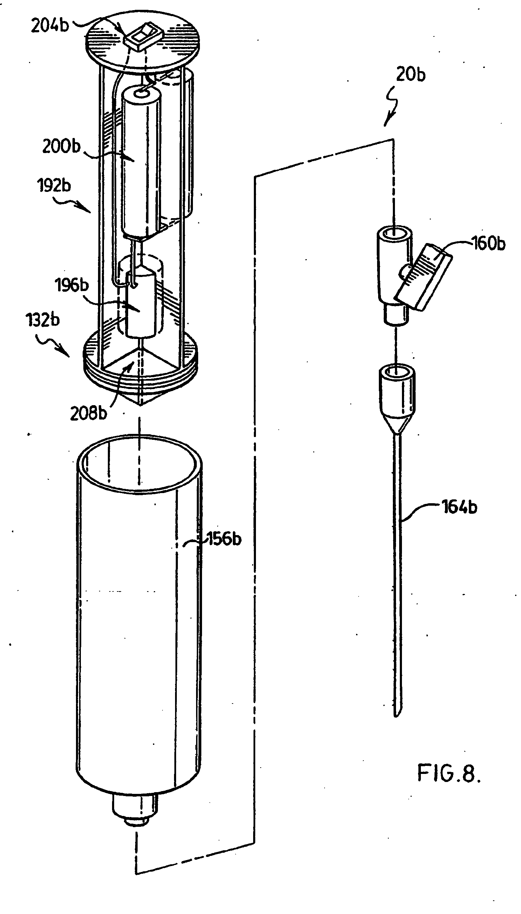

Referring now to Figure 8, an apparatus for administering a therapeutic agent into tissue according to an embodiment of the invention is indicated generally at 20b. Apparatus 20b is a self-contained, portable version of apparatuses 20 and 20a. Specifically, apparatus 20b includes a needle 164b and a two-way valve 160b that are substantially the same as needle 164a and valve 160a as described above. Apparatus 20b also includes a syringe 132b comprising a barrel 156b (or other chamber) and a plunger 192b (or other means to express the contents of the chamber). Plunger 192b is configured as a normal plunger on a syringe, but also includes an miniature ozone generator 196b mounted on the shaft of plunger 192b. In turn, ozone generator 196b is connected to a power supply 200b and an on-off switch 204b that is disposed on the exterior tip of the shaft of plunger 192b. A small channel 208b joins generator 196b to the opposite tip of the shaft of plunger 192b, such that when plunger 192b is disposed in barrel 156b, generator 196b is in communication with the interior of barrel 156b. In this manner, switch 204b can be activated and thereby cause ozone to be generated and fill barrel 156b. Of particular note, in the present embodiment ozone can be generated in either gaseous or liquid form.

While switch 204b is activated, it is generally desired to have valve 160b placed in the closed position to prevent ozone from flowing from barrel 156b into needle 164b. Once a sufficient or otherwise desired amount of ozone has been generated and filled barrel 156b, switch 204b is turned "off' to discontinue generation of ozone, and then apparatus 20b is used in much the same manner as administrator 32 as described above in relation to Figures 2 and 3. The ozone generator 196b can be provided in a variety of sizes, capable of delivering a range of ozone volumes, for example from about one cc to about five cc, but could be made to generate volumes of ozone liquid or gas form of about 0.1 cc to about one litre.

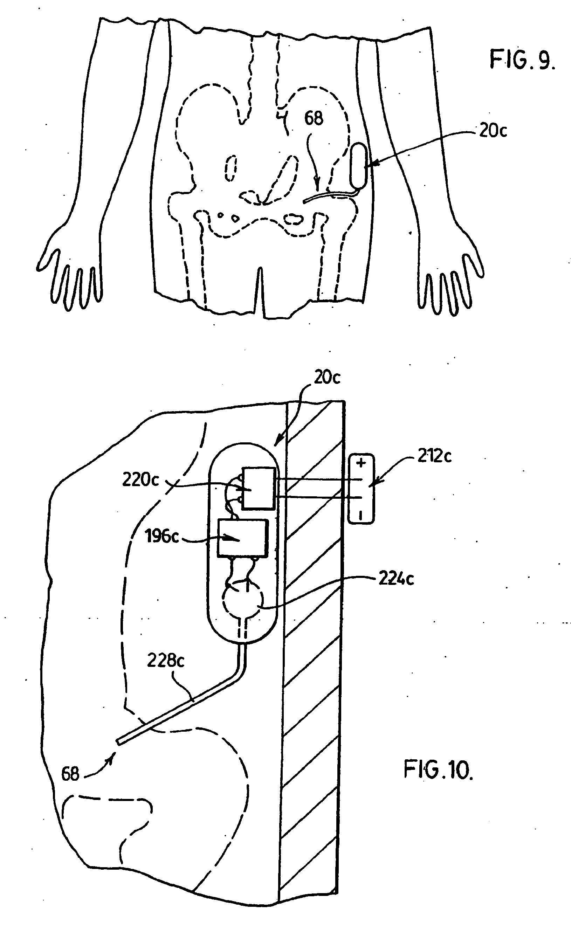

Referring now to Figures 9 and 10, an apparatus for administering a therapeutic agent into tissue is indicated generally at 20c. Apparatus 20c is a self-contained, implantable version of apparatuses 20, 20a and 20b. As shown in Figure 9, apparatus 20c is implanted subcutaneously proximal to hip 68. In a present embodiment, apparatus 20c includes a separate, external switchable power supply 212c that is disposed just above the skin of the patient and is thereby activatable on demand by the patient. Power supply 212c connects to a miniature ozone generator 196c disposed percutaneously. In turn, generator 196c is connected to a oxygen source 220c, such that when power supply 212c is "on", generator 196c will interact with source 220c of either oxygen or sterile water, to generate ozone in either in liquid or gaseous form, inside a cavity 224c. In turn, cavity 224c is connected to a catheter 228c, which interconnects with cavity 224c with the tissue inside hip 68 to which the ozone is being administered to relieve pain associated therewith.

As a variation of apparatus 20c, power supply 212c can be disposed subcutaneously, and a wireless switching means can be disposed on the outside of the patient, such as a magnetic switch or other types of wireless means for activating or deactivating the ozone generator. Advantageous to this technique is that the overall use of needles is diminished, and thus beam hardening artifacts that are generated by metallic objects that can prevent proper visualization of the disc details (as might occur when doing injections by means of image guidance, and as may be done in certain other embodiments herein,) are reduced. In sum, by reducing the use of needles, visualization is improved and allows the dorsal root ganglion to be clearly seen.

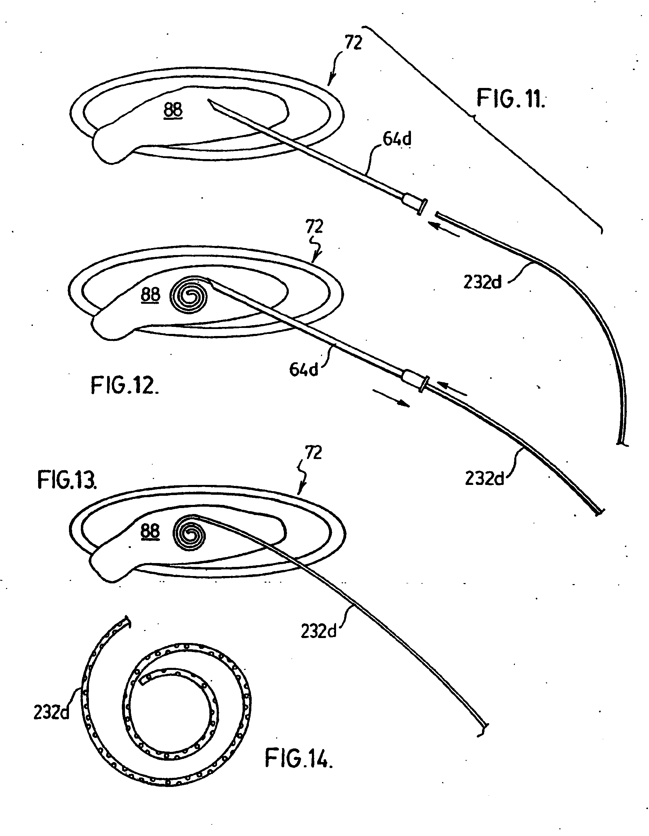

Referring now to Figures 11-14, in another variation an infusion wire 232d is passed through needle 64d until it coils inside nucleus pulposus 88 (or other tissue area for treatment.) Since infusion wire 232d is perforated along its length, as shown in Figure 14, once a desired or sufficient amount of wire 232d has been inserted into nucleus pulposus 88, an ozone source can be connected to the opposite end of the infusion wire 232d and injected into nucleus pulposus 88 via the infusion wire. Due to the perforations along infusion wire 232d, ozone is dispersed more readily into nucleus pulposus 88.

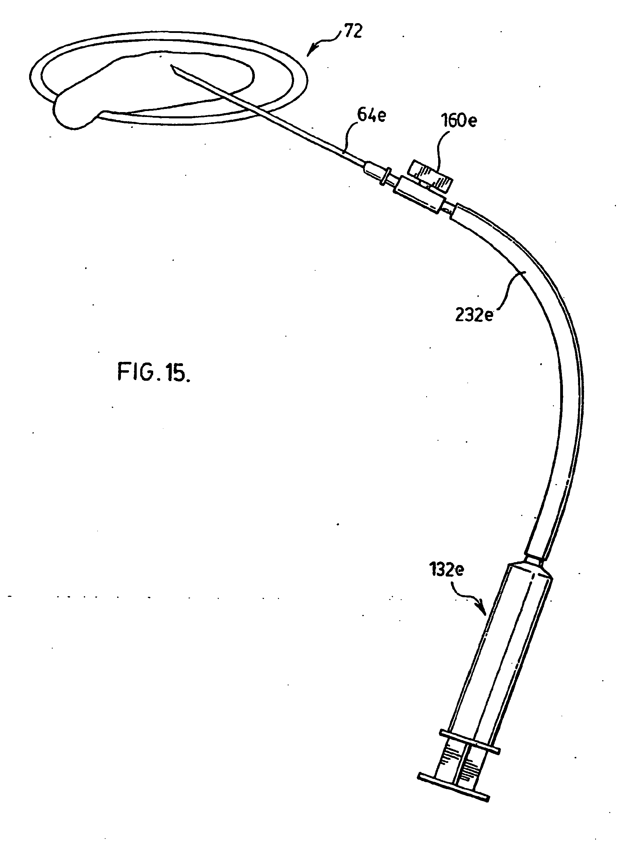

Referring now to Figure 15, in another variation it can be seen that various other configurations of how administration of the ozone are possible, include others within the scope of the invention when used with the claimed syringe design. Specifically, Figure 15 shows a syringe 132e connected to a valve 160e via tubing 232e.

A kit of parts for performing an injection of medical ozone for the treatment of a herniated disc or the like is possible. The kit includes a sterile tray with a number of compartments to hold
· a disinfectant, drape and skin preparation material,
· lidocaine and a 10 cc syringe with a 22-G. long needle,
· nonionic x-ray dye-for discogram such as Omnipaque 300 M,
· outer 16 or 18 G needle to act as the co-axial introducing needle,
· 20 or 22 G needle with possible side holes for injecting the ozone into the disc,
· outer large needle of 16-18 G for steroid injection (can also be used to inject ozone into the paraspinous soft tissue),
· an infusion wire for even distribution of the ozone into the disc · space
· a charcoal ozone scavenger (a bottle with charcoal and 100 % O₂),
· steroids and local anaesthetics contained in a vial, ready for injection,
· a source of ozone such as a generator, examples include the AOS-1M Medical Ozonator or AOS-1MS Stainless Medical Ozonator, or a disposable injector in which then ozone can be generated filled with ozone,
· a disposable filtered attachment for the syringe a one way stop cock attached to the ozone generator,
· connecting, non-compliant tubing of 20-30 cm in length to reduce radiation to the operator hands during injection under image guidance.
· a three-way stop cock linking the tubing to the charcoal ozone scavenger bottle
· a one-way stop cock linking the tubing to the syringe via the said disposable filtered adapter,
· pharmaceutical gel such as Povidone, and
· adhesive bandages with a gauze pad in the center, such as Band-aid, or small dressing.

It is also possible to provide a method consisting of first introducing the patient into the computer tomography ("CT") scanner and performing a diagnostic CT scan in order to identify the herniated disc such as disc 72. The CT gantry is subsequently draped and readied, the skin is prepared and local anaesthetic is applied to the skin and adjacent soft tissue. An 16-18 G needle is pinned down to the disc level and a 22 G needle is inserted into the disc. A discogram is performed to check symptoms by injection of x-ray dye. The ozone apparatus (in the form of any of the previously described apparatus or such other apparatus as may now occur to those of skill in the art.) is subsequently switched on and the oxygen/ozone concentration chosen. The oxygen/ozone mixtures to choose from are in the about 0µg/ml, about 10g/ml, about 20µg/ml, about 30µg/ml, about 40µg/ml or about 50µg/ml A presently preferred range is between about 10µg/ml and about 34µg/ml, but more preferably about 27µg/ml to about 28µg/ml. In other embodiments other gases or therapeutic agents can be used, such as pure oxygen if found to be therapeutically effective or desirable.

As a next step, the syringe is fitted with a disposable filtered attachment which is itself attached to 20-30 cm of non-compliant tubing fitted with a 3-way to cock on one end and a one-way stop cock on the other, such as that shown in Figure 15. Ozone gas is then aspirated into the 10-cc syringe (can also use a calibrated cardiac syringe) via the disposable filtered adapter and the connecting tubing such that the entire dead space is filled with a known concentration of ozone, The total volume of deadspace should be known so appropriate amounts of ozone are actually injected to the desired area. CT fluoro imaging or the like can be subsequently used to inject the ozone into the disc and paraspinous soft tissues. An infusion wire with a stiff" stylet could be used, as previously described. Once the ozone injection is completed, a stop cock on the connecting tubing is closed. All of the leftover ozone is injected into the charcoal scavenger or a pure oxygen tube/bottle attached to the connecting tubing via a Luer lock. Steroids are then injected through the coaxial outer large needle. Finally, all needles are removed, the skin is appropriately dressed and a bandage is used to cover the perforated skin.

It is also possible to provide the combined intradiscal and periganglionic injection of medical ozone and periganglionic injection of steroids which has an cumulative effect and enhances the overall outcome of treatment.

While only specific combinations of the various features and components of the present invention have been discussed herein, it will be apparent to those of skill in the art that desired subsets of the disclosed features and components and/or alternative combinations of these features and components can be utilized, as desired. For example, other types of pain can be treated using the teachings herein. For example, joints, tendons, ligaments are other areas that can be treated. Another example includes irrigating a wound site, such as a colostomy, with ozone to reduce pain at the wound site. As another example, an implantable device could put into the teeth (or other dental area) of a patient, similar to apparatus 20c to reduce pain in the dental region. As another example is to irrigate a subcutaneous pouch for holding a pacemaker or the like for sterilization and/or treatment of pain and/or decrease of inflammation and such other advantage corresponding to the therapeutic agent as will occur to those of skill in the art.

## Claims

1. A syringe (132b) for generation and administration of a therapeutic agent comprising:
a barrel (156b) for holding said agent;
a plunger (192b) for insertion into a first end of said barrel (156b) and expressing said agent from a second end of said barrel (156b);
a therapeutic agent generator (196b) integrally mounted coaxially with said plunger (192b) and in communication with said barrel (156b) through a channel 208b in said plunger (192b), said generator connected to a said power supply (200b); a switch (204b) connected to said power supply (200b) such that when said switch (204b) is activated said therapeutic agent generator (196b) generates said therapeutic agent and fills said barrel (156b) therewith
**characterised in that** the syringe provides self-contained generation and administration of said therapeutic agent and said power supply (200b) is integrally mounted coaxially with said plunger (192b).

2. The syringe (132b) of claim 1, wherein said agent is ozone.

3. The syringe (132b) of claim 1, wherein said agent is liquid or gaseous ozone.

4. The syringe (132b) of claim 1, wherein the syringe (132b) is made of polyethylene.

5. The syringe (132b) of claim 1, wherein the therapeutic agent generator (196b) is an ozone generator and is mounted on the shaft of the plunger (192b).

6. A syringe (132b) according to claim 1 in which the on-off switch (204b) for the p₆wer supply (200b) is disposed on the exterior tip of the shaft of the plunger (192b).

7. A syringe (132b) according to claim 1 in which said channel (208b) is small and joins the therapeutic agent generator (196b) to the opposing tip of the shaft of the plunger (192b) such that when the plunger (192b) is disposed in the barrel (156b) the therapeutic agent generator (196b) is in communication with the interior of the barrel (156b).

8. The syringe (132b) of claim 1, wherein a valve (160b) is provided between the barrel (156b) and a needle (164b) and the valve (160b) is in the closed position whilst the barrel (156b) is filled with ozone generated by the therapeutic agent generator (196b).

## Patentansprüche

1. Spritze (132b) zur Erzeugung und Verabreichung eines therapeutischen Mittels, umfassend:
einen Zylinder (156b) zum Aufnehmen des Mittels;
einen Kolben (192b) zur Einführung in ein erstes Ende des Zylinders (156b) und zum Herausdrücken des Mittels von einem zweiten Ende des Zylinders (156b);
einen Generator (196b) des therapeutischen Mittels, welcher koaxial mit dem Kolben (192b) und in Kommunikation mit dem Zylinder (156b) durch einen Kanal (208b) in dem Kolben (192b) integral montiert ist, wobei der Generator mit einer Stromversorgung (200b) verbunden ist; wobei ein Schalter (204b) derart mit der Stromversorgung (200b) verbunden ist, dass, wenn der Schalter (204b) aktiviert ist, der Generator (196b) des therapeutischen Mittels das therapeutische Mittel erzeugt und den Zylinder (156b) damit füllt,
**dadurch gekennzeichnet,**
**dass** die Spritze eine in sich abgeschlossene Erzeugung und Verabreichung des therapeutischen Mittels bereitstellt und die Stromversorgung (200b) koaxial mit dem Kolben (192b) integral montiert ist.

2. Spritze (132b) nach Anspruch 1, **dadurch gekennzeichnet, dass** das Mittel Ozon ist.

3. Spritze (132b) nach Anspruch 1, **dadurch gekennzeichnet, dass** das Mittel flüssiges oder gasförmiges Ozon ist.

4. Spritze (132b) nach Anspruch 1, **dadurch gekennzeichnet, dass** die Spritze (132b) aus Polyethylen hergestellt ist.

5. Spritze (132b) nach Anspruch 1, **dadurch gekennzeichnet, dass** der Generator (196b) des therapeutischen Mittels ein Ozongenerator ist und auf dem Schaft des Kolbens (192b) montiert ist.

6. Spritze (132b) nach Anspruch 1, **dadurch gekennzeichnet, dass** der An-AusSchalter (204b) für die Stromversorgung (200b) auf der äußeren Spitze des Schafts des Kolbens (192b) angeordnet ist.

7. Spritze (132b) nach Anspruch 1, **dadurch gekennzeichnet, dass** der Kanal (208b) klein ist und den Generator (196b) des therapeutischen Mittels mit der gegenüberliegenden Spitze des Schafts des Kolbens (192b) verbindet, so dass sich der Generator (196b) des therapeutischen Mittels in Kommunikation mit dem Inneren des Zylinders (156b) befindet, wenn der Kolben (192b) in dem Zylinder (156b) angeordnet ist.

8. Spritze (132b) nach Anspruch 1, **dadurch gekennzeichnet, dass** ein Ventil (160b) zwischen dem Zylinder (156b) und einer Nadel (164b) vorhanden ist, und dass sich das Ventil (160b) in der geschlossenen Stellung befindet während der Zylinder (156b) mit Ozon gefüllt wird, welches durch den Generator (196b) des therapeutischen Mittels erzeugt wird.

## Revendications

1. Seringue (132b) pour générer et administrer un agent thérapeutique comprenant :
un cylindre (156b) destiné à contenir ledit agent ;
un piston (192b) destiné à être inséré dans une première extrémité dudit cylindre (156b) et à exprimer ledit agent à partir d'une seconde extrémité dudit cylindre (156b) ;
un générateur d'agent thérapeutique (196b) monté d'un seul tenant de façon coaxiale avec ledit piston (192b) et en communication avec ledit cylindre (156b) par le biais d'un canal (208b) dans ledit piston (192b), ledit générateur étant relié à une source d'alimentation (200b) ; un interrupteur (204b) étant relié à ladite source d'alimentation (200b) de telle sorte que lorsque ledit interrupteur (204b) est actionné, ledit générateur d'agent thérapeutique (196b) génère ledit agent thérapeutique et en remplit ledit cylindre (156b),
**caractérisé en ce que** la seringue assure la génération et l'administration dudit agent thérapeutique et ladite source d'alimentation (200b) est montée d'un seul tenant de façon coaxiale avec ledit piston (192b).

2. Seringue (132b) selon la revendication 1, dans laquelle ledit agent est de l'ozone.

3. Seringue (132b) selon la revendication 1, dans laquelle ledit agent est de l'ozone liquide ou gazeux.

4. Seringue (132b) selon la revendication 1, dans laquelle la seringue (132b) est constituée de polyéthylène.

5. Seringue (132b) selon la revendication 1, dans laquelle le générateur d'agent thérapeutique (196b) est un générateur d'ozone et est monté sur l'axe du piston (192b).

6. Seringue (132b) selon la revendication 1, dans laquelle l'interrupteur marche-arrêt (204b) pour la source d'alimentation (200b) est disposé sur la pointe extérieure de l'axe du piston (192b).

7. Seringue (132b) selon la revendication 1, dans laquelle ledit canal (208b) est petit et relie le générateur d'agent thérapeutique (196b) à la pointe opposée de l'axe du piston (192b) de telle sorte que lorsque le piston (192b) est disposé dans le cylindre (156b), le générateur d'agent thérapeutique (196b) est en communication avec l'intérieur du cylindre (156b).

8. Seringue (132b) selon la revendication 1, dans laquelle un clapet (160b) est prévu entre le cylindre (156b) et une aiguille (164b) et le clapet (160b) est dans la position fermée alors que le cylindre (156b) est rempli avec de l'ozone généré par le générateur d'agent thérapeutique (196b).
